# EUROPEAN PATENT SPECIFICATION

(11) **EP 0 502 313 B1**
(45) Date of publication and mention of the grant of the patent: **16.07.1997**
(21) Application number: 92101515.2
(22) Date of filing: 30.01.1992
(51) Int. Cl.: A61K 38/06

(54) **Method for insuring adequate intracellular glutathione in tissue**
Verfahren zur Sicherstellung eines angemessenen intrazellulären Glutathionspiegels im Gewebe
Méthode pour assurer un taux de glutathione intracellulaire adéquat dans les tissus

(30) Priority: 04.02.1991 US 650222
(43) Date of publication of application: 09.09.1992
(73) Proprietor: SOCIETE DES PRODUITS NESTLE S.A., 1800 Vevey (CH)
(72) Inventor: Mark, David A., Oak Park, IL. 60304 (US); Rowe, W. Bruce, Evanston, IL. 60202 (US)
(74) Representative: Vuille, Roman

(56) References cited:
- EP-A- 0 357 776
- EP-A- 0 374 390
- FR-A- 2 544 613
- JOURNAL OF INFECTIOUS DISEASES vol. 144, no. 3, September 1981, CHICAGO page 281 BOUNOUS, G ET AL 'Influence of dietary lactalbumin hydrolysate on the immune system of mice and resistance to salmonellosis'
- J.Nutr., vol.125, pages 1462-1472, 1995

## Description

### BACKGROUND OF THE INVENTION

The present invention relates to compositions for use in clinical nutrition and more particularly to compositions for insuring adequate levels of intracellular glutathione.

It has been suggested that by increasing intracellular glutathione levels certain benefits can be realized. Glutathione protects cells against free radicals, reactive oxygen intermediates, and toxic compounds that are both of endogenous and of exogenous origin. Meister, "New Aspects of Glutathione Biochemistry and Transport-Selective Alteration of Glutathione Metabolism", Nutrition Review, 42:397-410.

The intracellular biosynthesis of glutathione is rate limited by cysteine. However, increasing cysteine per se is limited because of its instability, and the use of oxidized cystine is limited due to its low solubility.

It has been proposed to infuse into a patient a precursor that will stimulate the intracellular synthesis of glutathione. To this end, n-acetylcysteine, a precursor of cysteine that is involved in the intracellular synthesis of glutathione has been infused, parenterally, in patients to raise the glutathione level. It is also known to infuse, parenterally, L-2-oxothiazolidine-4-carboxylate and glutathione esters to also stimulate intracellular glutathione production. See, U.S. Patent Nos. 4,335,210; 4,434,158; 4,438,124; 4,647,571; 4,665,082; and 4,784,685.

Although the parenteral infusion of cysteine precursors as well as glutathione esters is believed to be an effective way to increase or maintain a sufficient level of intracellular glutathione, it would of course be desirable if the intracellular glutathione level could be maintained or increased through an enteral diet. This is especially true in view of the fact that a number of chronic disease states exhibit reduced or below normal cellular glutathione levels. One of the difficulties in increasing through an enteral regimen intracellular glutathione levels is that it is not typically possible merely to provide an enteral amino acid solution rich in cysteine. Cysteine typically will crystallize out as cystine in solution, e.g., an amino acid solution. Cystine is not readily biologically available to cells. Therefore, cysteine is not biologically available as a pharmaceutical.

Chung, et al., L-2-oxothiazolidine-4-carboxylate As A Cysteine Precursor: Efficacy for Growth and Hepatic Glutathione Synthesis in Chicks and Rats, American Institute of Nutrition (1990), sets forth experiments, by which they conclude, that orally administered L-2-oxothiazolidine-4-carboxylate is active as a cysteine precursor. In the experiments, they also administered, enterally, L-cysteine-HCl-H₂O. The paper also noted that "[i]t is well established that Cys [cysteine] is toxic when it is provided in excess. Rats given Cys via intraperitoneal injection show signs of toxicity, whereas those given OTC (on an equimolar basis to Cys) do not."

European published patent application 0 374 390 discusses a whey protein composition comprising a suitable concentrate that contains proteins in an essentially undenatured state. The application states that:
"It was shown, in controlled experiments, for the first time, that whey protein feeding of mice specifically enhances the immune response to sheep red blood cells (SRBC) and their resistance to pneumococcal infection, inhibits the development of DMH-induced colon cancer and increases tissue glutathione (GSH) levels independently of its nutritional quality.
   The present invention shows the correlation between the undenatured conformation of whey protein concentrate (w.p.c.) and host immunoenhancement whereby chemical indices of denaturation are given and the demonstration that the same crucial role of molecular conformation (undenatured state) applies to GSH promotion, which is the other major biological activity of w.p.c.
   Equally important is the demonstration that another protein source such as egg white, with the same high cysteine content as w.p.c. does not enhance GSH synthesis, further demonstrating the specificity of w.p.c. with respect to the described biological activity.
   The GSH promoting activity of undenatured w.p.c. is sustained over time (3 - 4 months).
   Whey and whey protein have been utilized from time immemorable for nutritional purposes. In addition, whey was recommended in folk and ancient medicine for the treatment of various diseases^{(1,2)} and, in one instance, lifetime feeding of hamsters with a whey protein diet has been shown to promote longevity with no explanation given^{(3,4)}.
   All these conditions appear to be somehow related to changes in glutathione which is a ubiquitous element exerting a protective effect against superoxide radicals and other toxic agents." (See page 2, lines 17-34.)

The European application also states that the biological activity of the whey protein "is actually dependent on the undenatured conformation of the proteins" (see page 13, lines 21-23). Additionally, the European application states that "the administration of glutathione itself is of little consequence on tissue glutathione levels, because it apparently cannot be transported intact across the cell membrane" (see page 6, lines 23-24).

The ability to give a composition orally that increases intracellular glutathione is highly desirable. However, all patient populations in need of same may not exhibit the same characteristics especially with respect to the bioavailability of the product to the patient. For patients having reduced or compromised gut function undenatured whey may not be absorbed in sufficient quantities so as to be readily bioavailable. Such patients with reduced gut function can include patients who suffer from: acquired immune deficiency syndrome (AIDS); Crohn's disease; chronic inflammatory bowel disease; short bowel syndrome; and inflammatory bowel reaction to radiation therapy. Not only do such patients typically have a reduced gut function, but, they may have reduced intracellular glutathione levels that should be elevated.

### SUMMARY OF THE INVENTION

The present invention provides a composition for insuring adequate intracellular glutathione levels in tissue. Pursuant to the present invention, a composition for increasing intracellular glutathione levels in patients having compromised gut function is provided comprising a therapeutically effective amount of a solution including hydrolyzed proteins enriched in cysteine.

The composition can be administered enterally.

In an embodiment of the invention, the solution includes hydrolyzed whey.

In an embodiment of the invention, the solution includes hydrolyzed egg white protein.

In an embodiment, the present invention includes a method for increasing intracellular glutathione levels in a patient having compromised gut function comprising enterally administering to the patient a therapeutically effective amount of a solution including hydrolyzed whey or egg white protein.

In an embodiment, a method for treating a patient having compromised gut function is provided comprising the steps of administering enterally to the patient a sufficient amount of a hydrolyzed protein including cysteine and an amount of glutathione to increase the intracellular glutathione levels of the patient.

Additional features and advantages of the present invention are described in, and will be apparent from, the detailed description of the presently preferred embodiments.

### DETAILED DESCRIPTION OF THE PRESENTLY PREFERRED EMBODIMENTS

The present invention provides a composition for insuring adequate intracellular glutathione levels in tissue. Additionally, the present invention provides a composition for increasing intracellular glutathione levels in a patient in need of same, specifically a patient having compromised gut function.

The composition, in an embodiment, is an enterally administered therapeutically effective amount of a solution including hydrolyzed proteins enriched in cysteine. Because the proteins are hydrolyzed, they are readily absorbed, even in a patient having compromised gut function.

In another embodiment, the invention comprises administering to a patient a therapeutically effective amount of a solution including hydrolyzed protein and an amount of glutathione.

The inventors have found, that by administering a hydrolyzed protein including a sufficient amount of cysteine, that it is possible not only to maintain sufficient levels of glutathione in patients having compromised gut function and in need of increased glutathione levels, indeed, it is possible to increase the intracellular glutathione levels in these patients who have a depressed level of glutathione.

An advantage of present invention is that for a patient who has compromised gut function, it is possible to administer enterally the composition of the present invention and provide a sufficient level of intracellular glutathione. In fact, it is possible to increase the intracellular glutathione level to normal levels.

Typically, for example, an immune-compromised patient has below normal cellular levels of glutathione. It is believed that a patient who has decreased glutathione levels is more susceptible to many disease states. It is therefore important to insure that cellular glutathione levels are maintained at near normal levels, or increased to meet those levels.

A number of such patients having reduced glutathione levels also have impaired or compromised gut functions. Examples of such patients include those suffering from: acquired immune deficiency syndrome (AIDS); Crohn's disease; chronic inflammatory bowel disease; short bowel syndrome; and inflammatory bowel reaction to radiation therapy. Due to the compromised gut function providing an undenatured protein, such as whey would not provide a bioavailable source of glutathione to the patient. By providing, in an embodiment, hydrolyzed protein enriched in cysteine, it is possible to insure adequate glutathione levels in such patients.

In an embodiment of present invention, hydrolyzed whey protein or hydrosate egg white protein is administered to the patient. Whey protein and egg white protein are enriched in cysteine. In a preferred embodiment, at least partially hydrolyzed whey or egg white protein is administered to the patient.

In an embodiment of the present invention, whey protein hydrolysate is administered to a patient. An example of an enteral diet that can be administered to the patient is Peptamen®, Clintec Nutrition Company, Deerfield, Illinois. Peptamen® includes whey protein hydrolysate. The peptide distribution of Peptamen® is as follows:

| **Peptide Size:** (number of amino acid residues) | Approximate molecular weight | Percentage by number |
|---|---|---|
| 1 (free amino acid) | 133 | 12% |
| 2-4 | 100-500 | 17% |
| 5-9 | 500-1000 | 38% |
| 10-40 | 1000-4600 | 28% |
| >40 | >4600 | 5% |
| Average peptide size: 8 amino acid residues | | |

The nutrient composition of Peptamen® Liquid Elemental Diet is as follows:

| **NUTRIENT INFORMATION** Serving Size One Can (500 ml) | | | | | |
|---|---|---|---|---|---|
| NUTRIENT COMPOSITION | | PER 500 ml | | PER 2000ml | |
| | | AMOUNT | %U.S.RDA | AMOUNT | %U.S.RDA |
| CALORIES | kcal | 500 | ** | 2000 | ** |
| PROTEIN | g | 20.0 | 44 | 80 | 178 |
| CARBOHYDRATE | g | 63.5 | ** | 254 | ** |
| FAT* | g | 19.5 | ** | 78 | ** |

| Vitamin Composition | | | | | |
|---|---|---|---|---|---|
| VITAMIN A | IU | 1875 | 37 | 7500 | 150 |
| VITAMIN D | IU | 100 | 25 | 400 | 100 |
| VITAMIN E | IU | 10 | 33 | 40 | 133 |
| VITAMIN K | mcg | 62.5 | ** | 250 | ** |
| VITAMIN C | mg | 50 | 83 | 200 | 333 |
| THIAMINE (B¹) | mg | 0.75 | 50 | 3 | 200 |
| RIBOFLAVIN (B₂) | mg | 0.85 | 50 | 3.4 | 200 |
| NIACIN | mg | 10 | 50 | 40 | 200 |
| VITAMIN B₆ | mg | 1.5 | 75 | 6 | 300 |
| FOLIC ACID | mcg | 200 | 50 | 800 | 200 |
| PANTOTHENIC ACID | mg | 5 | 50 | 20 | 200 |
| VITAMIN B₁₂ | mcg | 3 | 50 | 12 | 200 |
| BIOTIN | mcg | 150 | 50 | 600 | 200 |
| CHOLINE | mg | 225 | ** | 900 | ** |

Additionally, glutathione can be administered with the hydrolyzed protein to further assist in increasing intracellular glutathione levels. The glutathione can be added to the hydrolyzed protein that is enriched in cysteine.

In an embodiment, approximately 1 to about 2.8 grams of glutathione is added per liter of Petamen®.

By way of example, but not limitation, contemplative examples of the present invention will now be given.

### Contemplative Example 1

A 32-year-old white male has been known to be HIV positive for seven years. He was essentially free of symptoms until two years ago when he had a complicated medical course following an upper respiratory tract infection. During the course of this illness he lost approximately 15% of his usual body weight. Following recovery from this illness, he was relatively free of symptoms except for occasional episodes of diarrhea of unknown etiology. Despite dietary and nutritional counseling he was unable to regain significant weight due, in part, to compromised gut function.

The use of a hydrolyzed whey protein dietary supplement was recommended to aid in increasing nutrient intake and to aid in absorption of nutrients. He was able to drink approximately one liter of this supplement increasing his calorie intake by approximately 1000 kcal per day and to increase his protein intake by 40 grams per day.

He gained six pounds in the first two weeks of dietary supplementation and continued to gain two to three pounds between biweekly clinic visits despite somewhat reduced intake of the supplement. He remained symptom-free and has had no further episodes of diarrhea.

Plasma glutathione and cysteine levels were found to be at the levels seen in normal healthy individuals at 4 weeks following initiation of supplementation.

### Contemplative Example 2

A 54-year-old white male has been known to be HIV positive for five years. In the last six months he has had several opportunistic infectious episodes including two lung infections that required hospitalization. During the second hospitalization he was placed on total parenteral nutrition (TPN) for eight days to increase nutrient intake and restore a significant weight loss (18% of usual body weight). Before discharge from the hospital he was transitioned to oral intake using an enteral feeding formula based on hydrolyzed whey protein. He continued to use the preparation as an occasional dietary supplement after release from the hospital.

During the period when he received TPN, a plasma amino acid profile was performed. Among several deviations from the normal amino acid profile was a 90% reduction in plasma cysteine concentration. This observation was followed by an analysis of plasma glutathione and white blood cell glutathione concentrations. Both plasma and white blood cell glutathione concentrations were less than 10% of normal values.

These analyses were repeated one week after hospital discharge following two weeks of oral intake of the enteral supplement. At this time both plasma cysteine and glutathione were at the low end of normal values. White blood cell glutathione levels were at approximately 60% of normal values.

## Claims

1. Use of a whey or egg white protein hydrolysate enriched in cysteine for the preparation of an enteral composition for increasing intracellular glutathione levels in a patient having reduced gut function.

2. Use of whey or egg white protein hydrolysate enriched in cysteine and a therapeutically effective amount of glutathione for the preparation of an enteral composition for increasing intracellular glutathione levels in a patient having reduced gut function.

3. The use according to claims 1 or 2 wherein the patient has reduced gut function as a result of an immune deficiency, Crohn's disease, inflammatory bowel disease, short bowel syndrome, or inflammatory bowel reaction to irradiation.

## Patentansprüche

1. Verwendung von Molken- oder Eiklar-Proteinhydrolysat, das bezüglich Cystein angereichert ist, zur Herstellung einer enteralen Zusammensetzung zur Erhöhung der intrazellulären Glutathionspiegel eines Patienten mit verminderter Darmfunktion.

2. Verwendung von Molken- oder Eiklar-Proteinhydrolysat, das bezüglich Cystein und einer therapeutisch wirksamen Menge an Glutathion angereichert ist, zur Herstellung einer enteralen Zusammensetzung zur Erhöhung der intrazellulären Glutathionspiegel eines Patienten mit verminderter Darmfunktion.

3. Verwendung nach Anspruch 1 oder 2, bei der der Patient an einer verminderten Darmfunktion infolge eines Immunmangels, der Crohn Krankheit, einer entzündlichen Darmerkrankung, des Short-bowel-Syndroms oder einer entzündlichen Darmreaktion auf Bestrahlung leidet.

## Revendications

1. Utilisation d'un hydrolysat de protéine de petit lait ou de blanc d'oeuf enrichi en cystéine pour la préparation d'une composition entérale destinée à accroître les taux de glutathion intracellulaire chez un patient présentant une fonction digestive réduite.

2. Utilisation d'un hydrolysat de protéine de petit lait ou de blanc d'oeuf enrichi en cystéine et d'une quantité thérapeutiquement efficace de glutathion pour la préparation d'une composition entérale destinée à accroître les taux de glutathion intracellulaire chez un patient présentant une fonction digestive réduite.

3. Utilisation suivant la revendication 1 ou 2, dans laquelle le patient présente une fonction digestive réduite en résultat d'une déficience immunitaire, de la maladie de Crohn, d'une maladie intestinale inflammatoire, d'une malabsorption par résection intestinale étendue ou d'une réaction intestinale inflammatoire à une irradiation.
